# EUROPEAN PATENT APPLICATION

(11) **EP 1 123 923 A1**
(43) Date of publication of application: **16.08.2001**
(21) Application number: 99949368.7
(22) Date of filing: 22.10.1999
(51) Int. Cl.: C07D 211/90, A61K 31/496, A61P 9/10, A61P 25/16, A61P 25/28, A61P 25/04, A61P 25/06, A61P 25/30, A61P 11/06, A61P 1/00

(54) **DIHYDROPYRIDINE DERIVATIVES AND DRUG COMPOSITIONS CONTAINING THE SAME**

(30) Priority: 23.10.1998 JP 30306698
(71) Applicant: Ajinomoto Co., Inc., Tokyo 104-0031 (JP)
(72) Inventor: NIWA, Seiji, Ctrl Res. Lab., Ajinomo Co. Inc., Kawasaki-shi, Kanagawa 210-0801 (JP); OHNO, Seiji, Ctrl Res. Lab. Ajinomo Co. Inc., Kawasaki-shi, Kanagawa 210-0801 (JP); TAKAHARA, Akira, Ctrl Res. Lab. Ajinomo Co. Inc., Kawasaki-shi, Kanagawa 210-0801 (JP); KITO, Morikazu, Ctrl Res. Lab., Ajinomo Co. Inc., Kawasaki-shi, Kanagawa 210-0801 (JP)
(74) Representative: Nicholls, Kathryn Margaret
(86) International application number: JP9905840
(87) International publication number: WO0024716

(57) **Abstract**

Dihydropyridine derivatives represented by the following formula: 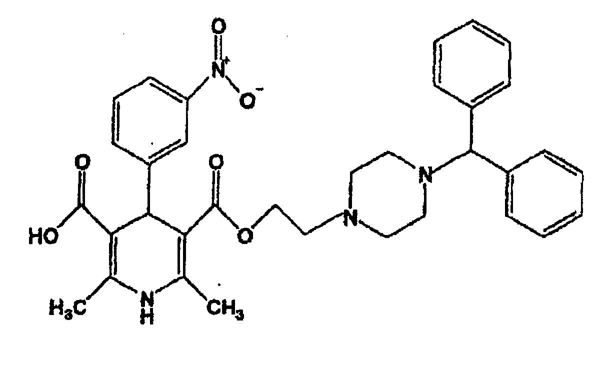 analogs thereof and pharmaceutically acceptable salts thereof are provided. These compounds are usable as N-type calcium channel antagonists, particularly therapeutic agents for various diseases such as acute stage of ischemic cerebrovascular disorders caused by cerebral infarction or intracerebral bleeding, and Alzheimer's disease.

## Description

### Background of the Invention

The present invention relates to new dihydropyridine derivatives and the use of the dihydropyridine derivatives as medicines. The activation of N-type calcium channel is concerned with various diseases, for example, acute stage of ischemic cerebrovascular disorders caused by cerebral infarction or intracerebral bleeding (including subarachnoidal hemorrhage); progressive neurodegenerative diseases such as Alzheimer's disease, AIDS related dementia and Parkinson's disease, dementia due to cerebrovascular disorder and ALS; neuropathy caused by head injury; various pains such as pain caused by spinal injury, diabetes or thromboangiitis obliterans, postoperative pain, migraine and visceral pain; various diseases associated with psychogenic stress such as bronchial asthma, unstable angina and irritable colitis; emotional disorder and withdrawal symptoms after addiction to drugs such as ethanol withdrawal symptoms. The compounds of the present invention can inhibit the activation of the N-type calcium channel and, therefore usable as remedies for these diseases.

Calcium channels are now classified into subtypes of L, N, P, Q, R and T. Each subtype of calcium channels is organ-specifically distributed. It is known that particularly N-type calcium channel is widely distributed in pars centralis, peripheral nerves and adrenomedullary cells and participates in neuronal cell death, regulation of blood catecholamine level and control of senses such as perception.

It has been confirmed that omega conotoxin GVIA and omega conotoxin MVIIA, which are peptides selectively inhibiting N-type calcium channel, inhibit the release of excitatory neurotransmitters in the sliced brain preparation. It is also confirmed in animal experiments that they inhibit the progress of neuronal necrosis associated with cerebrovascular disorders. It is generally considered that compounds having a N-type calcium channel blocking action are clinically effective in the treatment of acute stage of ischemic cerebrovascular disorders caused by cerebral infarction or intracerebral bleeding (including subarachnoidal hemorrhage); progressive neurodegenerative diseases such as Alzheimer's disease, AIDS related dementia and Parkinson's disease, dementia due to cerebrovascular disorder and ALS; and neuropathy caused by head injury. Further, it is confirmed in animal tests that omega conotoxin MVIIA relieves a pain induced by formaldehyde, hot plate and peripheral neuropathy. Accordingly, omega conotoxin MVIIA is considered to be clinically effective against various pains such as pain caused by spinal injury, diabetes or thromboangiitis obliterans, postoperative pain, migraine and visceral pain. In addition, because omega conotoxin GVIA inhibits the release of catecholamine from cultured sympathetic ganglion cells, catecholamine secretion from canine adrenal medulla and the contraction of the isolated blood vessel by electric stimulation of the perivascular nerve, it is considered that compounds having N-type calcium channel-blocking effects are clinically effective against various diseases related to psychogenic stress such as bronchial asthma, unstable angina and irritable colitis [Neuropharmacol., 32, 1141 (1993)].

Some peptidergic and non-peptidergic compounds which selectively affect N-type calcium channels have been ever disclosed (see, for example, WO 9313128). However, none of them was actually used as a medicine. Some of the compounds which affect N-type calcium channels are also effective against various types of calcium channels of other than N-type [British Journal of Pharmacology, 122 (1) 37-42, 1997]. For example, compounds having an antagonistic effect on L-type calcium channels which are very closely related to hypotensive effect, could not be used for diseases for which N-type antagonists will be used (such as cerebral stroke, neuralgia, terminal cancer pain and pain of spinal injury).

### Disclosure of the Invention

The object of the present invention is to provide new compounds having a selective antagonistic effect on N-type calcium channels.

Another object of the present invention is to provide antagonists to N-type calcium channel.

Still another object of the present invention is to provide a therapeutic agent for any of acute stage of ischemic cerebrovascular disorders caused by cerebral infarction or intracerebral bleeding, Alzheimer's disease, AIDS related dementia, Parkinson's disease, progressive neurodegenerative diseases, neuropathy caused by head injury, pain caused by thromboangiitis obliterans, postoperative pain, migraine, visceral pain, bronchial asthma, unstable angina, irritable colitis and withdrawal symptoms after addiction to drugs.

The above-described objects and other objects of the present invention will be apparent from the following description and Examples.

After synthesizing various dihydropyridine derivatives and examining the N-type calcium current inhibiting effect of the newly synthesized compounds and well-known dihydropyridine derivatives for the purpose of solving the above-described problems, the inventors have found that specified, new dihydropyridine derivatives have an excellent effect of selectively antagonizing N-type calcium channel. The present invention has been completed on the basis of his finding.

Namely, the present invention provides dihydropyridine derivatives of the following general formula (1) and pharmaceutically acceptable salts thereof. 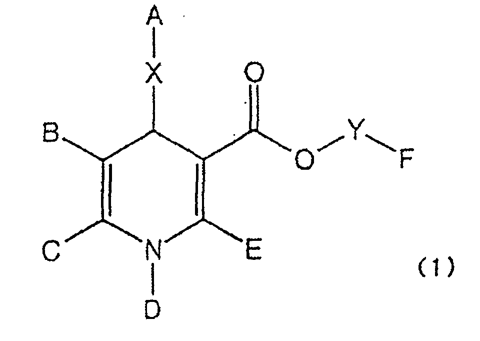 wherein A represents a group of the following general formula (2), or 1-naphthyl, 2-naphthyl, thiophene-3-yl, thiophene-2-yl, furan-3-yl, furan-2-yl, pyridine-4-yl, pyridine-3-yl, pyridine-2-yl, indole-2-yl, indole-3-yl, quinoline-2-yl, quinoline-3-yl, quinoline-4-yl, quinoline-5-yl, quinoline-6-yl, quinoline-7-yl, quinoline-8-yl, cyclohexyl or cyclopentyl group: 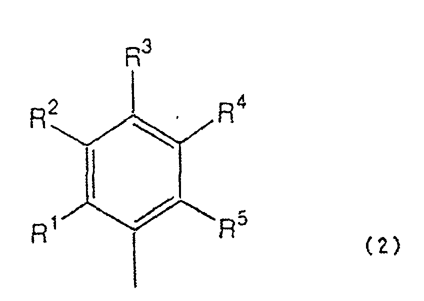 wherein R¹, R², R³, R⁴ and R⁵ may be the same or different from each other and each represent a hydrogen atom, halogen atom, hydroxyl group, carboxyl group, amino group, cyano group, nitro group, a lower alkyl group, a lower alkoxyl group, a lower alkenyl group, a lower alkynyl group, a lower alkylamino group, a lower alkylthio group, a lower alkanoyl group, a hydroxy-lower alkyl group, a hydroxy-lower alkoxyl group, a hydroxy-lower alkenyl group, a halogeno-lower alkyl group, a halogeno-lower alkoxyl group, a halogeno-lower alkenyl group, an aryl-lower alkoxyl group, a lower-alkoxycarbonyl group, an aryl group, a heteroaryl group or an aroyl group,
B represents carbamoyl group, cyano group, nitro group, acetyl group or carboxyl group,
C represents a hydrogen atom, methyl group, ethyl group or dimethoxymethyl group,
D represents a hydrogen atom, a lower alkyl group, a hydroxy-lower alkyl group or an aryl-lower alkyl group,
E represents a hydrogen atom, methyl group, ethyl group, dimethoxymethyl group or cyano group,
F represents a heterocyclic group or a cycloalkyl group,
X represents an interatomic bond, -CH₂-, -CH₂CH₂-, -CH=CH- or -C≡C-, and
Y represents an interatomic bond, -CH₂- or a group of any of the following general formulae (3) to (15): 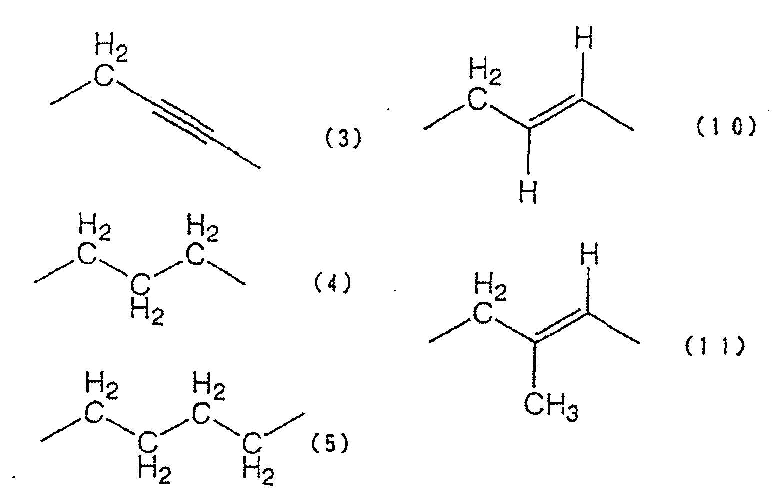 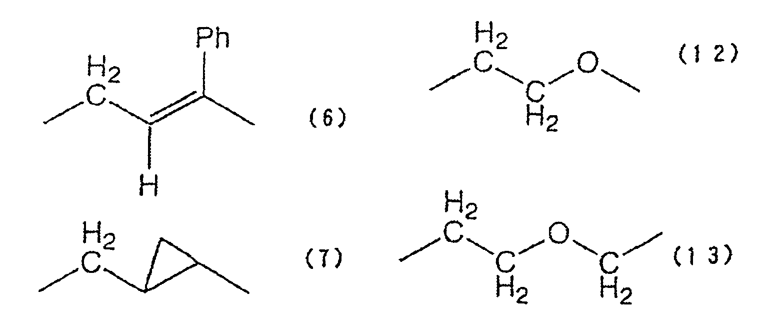 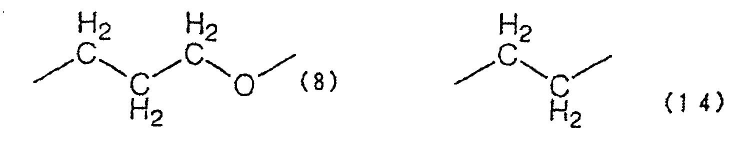 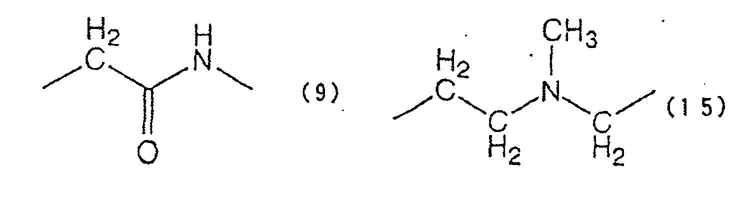
with the proviso that when Y represents any of the groups of general formulae (3) to (15), the heterocyclic groups represented by F exclude groups of the following general formula (16), cyclohexyl group, thiophene-3-yl group, thiophene-2-yl group, furan-3-yl group, furan-2-yl group, pyridine-4-yl group, pyridine-3-yl group and pyridine-2-yl group: 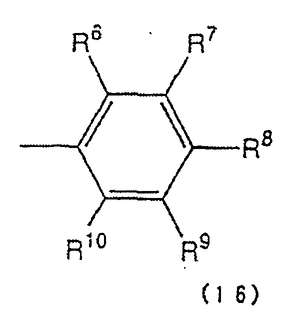 wherein R⁶, R⁷, R⁸, R⁹ and R¹⁰ may be the same or different from each other, and each represent hydrogen atom, a halogen atom, hydroxyl group, carboxyl group, amino group, cyano group, nitro group, a lower alkyl group, a lower alkoxyl group, a lower alkenyl group, a lower alkynyl group, a lower alkylamino group, a lower alkylthio group, a lower alkanoyl group, a hydroxy-lower alkyl group, a hydroxy-lower alkoxyl group, a hydroxy-lower alkenyl group, a halogeno-lower alkyl group, a halogeno-lower alkoxyl group, a halogeno-lower alkenyl group, an aryl-lower alkoxyl group, a lower-alkoxycarbonyl group or an aroyl group, and two of R¹ through R³ in general formula (2) may be bonded to each other to form a ring.

The present invention also provides N-type calcium channel antagonist containing a dihydropyridine derivative of above general formula (1) or a pharmaceutically acceptable salt thereof as an active ingredient.

The present invention further provides a therapeutic agent containing the above-described dihydropyridine derivative or a pharmaceutically acceptable salt thereof as the active ingredient, for any of acute stage of ischemic cerebrovascular disorders caused by cerebral infarction or intracerebral bleeding, Alzheimer's disease, AIDS related dementia, Parkinson's disease, progressive neurodegenerative diseases, dementia due to cerebrovascular disorder, pain caused by thromboangiitis obliterans, postoperative pain, migraine, visceral pain, bronchial asthma, unstable angina, irritable colitis and withdrawal symptoms after addiction to drugs.

The present invention also provides a pharmaceutical composition containing the dihydropyridine derivative or a pharmaceutically acceptable salt thereof and a carrier and/or a diluent.

### Best Mode for Carrying Out the Invention

The term "lower" herein indicates that the group has 1 to 6 carbon atoms. Alkyl groups themselves and also alkyl groups in alkoxyl groups, alkenyl groups, alkylamino groups, alkylthio groups and alkanoyl groups may be either linear or branched. Examples of these alkyl groups are methyl group, ethyl group, propyl group, isopropyl group, butyl group and secondary and tertiary butyl groups. Among them, those having 1 to 3 carbon atoms are preferred. The aryl-lower alkoxyl groups include, for example, benzyloxy group. The halogen atoms include fluorine, chlorine, bromine and iodine atoms. The aryl groups are both substituted and unsubstituted aryl groups. They are preferably phenyl group and substituted phenyl group, and the substituents are particularly halogens, alkyl groups and alkoxyl groups. Examples of the aroyl groups include benzoyl group and pyridylcarbonyl group.

The heterocyclic groups in the present invention may have a substituent. The substituents are, for example, halogen atoms, alkyl groups, alkanoyl groups, aryl groups, arylalkyl groups, alkoxyl groups, nitro group and cyano group.

The heterocyclic group F in the compounds of general formula (1) of the present invention is preferably pyrrolidine, piperazine, pyrazolidine, imidazolidine, tetrahydrofuran, tetrahydropyran, dioxane, tetrahydrothiophene, morpholine, imidazole, pyrrolidinone, oxazole, isoxazole, pyrimidine, pyrazine, pyridazine or piperidine group.

R¹, R², R³, R⁴ and R⁵ in general formula (2), which may be the same or different from each other, are preferably hydrogen atom, a halogen atom, hydroxyl group, carboxyl group, cyano group, nitro group, a lower alkyl group, a lower alkoxyl group, a halogeno-lower alkyl group or a lower-alkoxycarbonyl group. Preferably D represents hydrogen atom, X represents an interatomic bond, Y represents an interatomic bond, methylene group, ethylene group or propylene group, and B represents carboxyl group.

It is more preferred that R¹, R², R³, R⁴ and R⁵ in general formula (2), which may be the same or different from each other, are hydrogen atom, a halogen atom, carboxyl group, cyano group, nitro group or a halogeno-lower alkyl group, C represents methyl group, E represents methyl group and F represents any of pyrrolidine group, piperazine group, imidazole group, pyrrolidinone group and piperidine group.

It is more preferred that A is represented by general formula (2), wherein R¹, R³, R⁴ and R⁵ each represent hydrogen atom and R² represents nitro group, C represents methyl group, E represents methyl group and F represents piperidine group or piperazine group.

It is particularly preferred that A is represented by general formula (2), wherein R¹, R³, R⁴ and R⁵ each represent hydrogen atom and R² represents nitro group, B represents carboxyl group, C represents methyl group, D represents hydrogen atom, E represents methyl group, F represents piperidine group or piperazine group, X represents an interatomic bond, and Y represents any of interatomic bond, methylene group, ethylene group and propylene group.

Dihydropyridine derivatives (1) of the present invention can be produced by processes described below:

For example, dihydropyridine derivatives (1-1) wherein D represents hydrogen atom and B represents carboxyl group can be produced by the following reaction scheme: 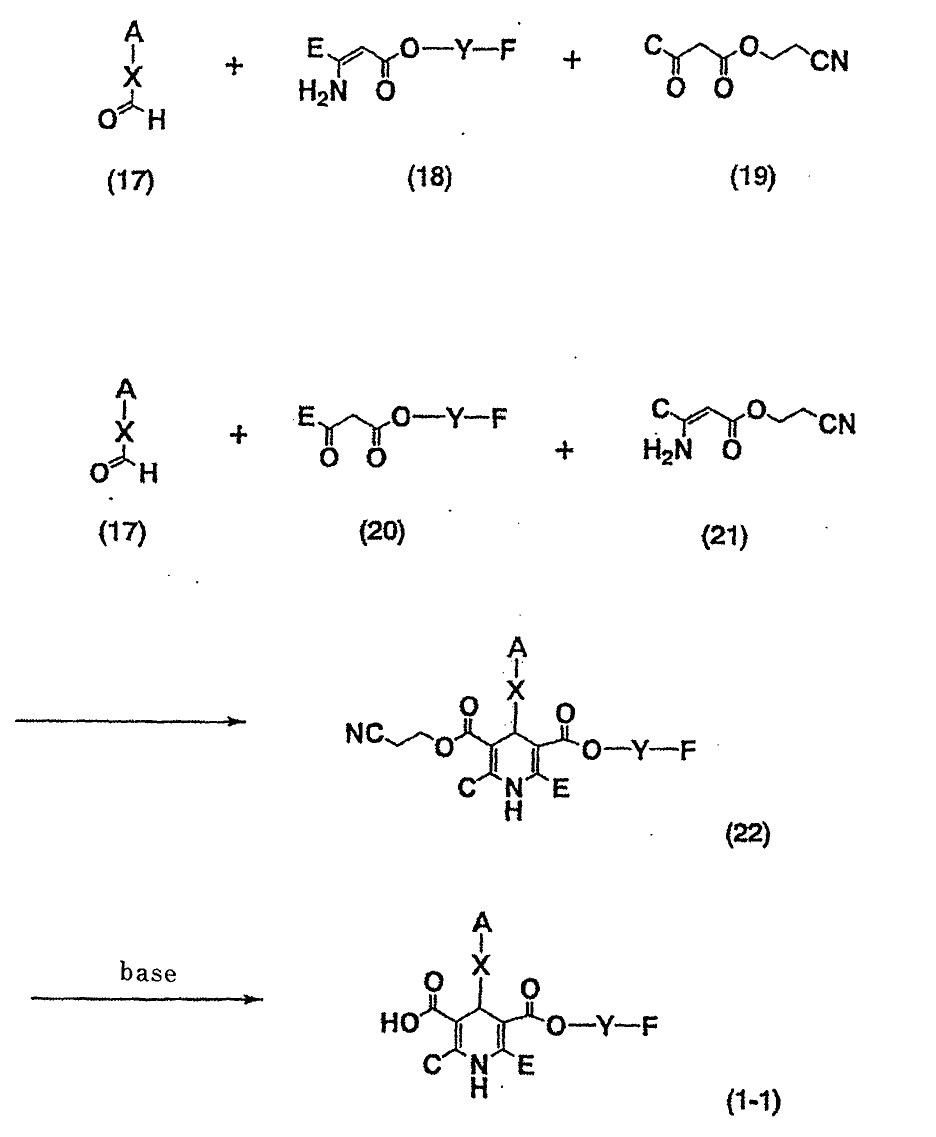 wherein A, C, E, F, X and Y are as defined above.

Namely, a dihydropyridinedicarboxylic acid diester (22) can be obtained by reacting an aldehyde (17) with a 3-aminocrotonic acid ester (18) having a substituent E at the 3-position and 2-cyanoethyl ester of ketocarboxylic acid (19) or by reacting the aldehyde (17) with a ketoester (20) and 2-cyanoethyl ester of 3-aminocrotonic acid (21) having a C substituent at the 3-position. The dihydropyridinecarboxylic acid derivatives (1-1) of the present invention can be produced by treating the obtained diester of dihydropyridinedicarboxylic acid with a base such as sodium hydroxide.

In another process, dihydropyridinecarboxylic acid derivatives (1-1) of the present invention can be produced by the following reaction scheme:

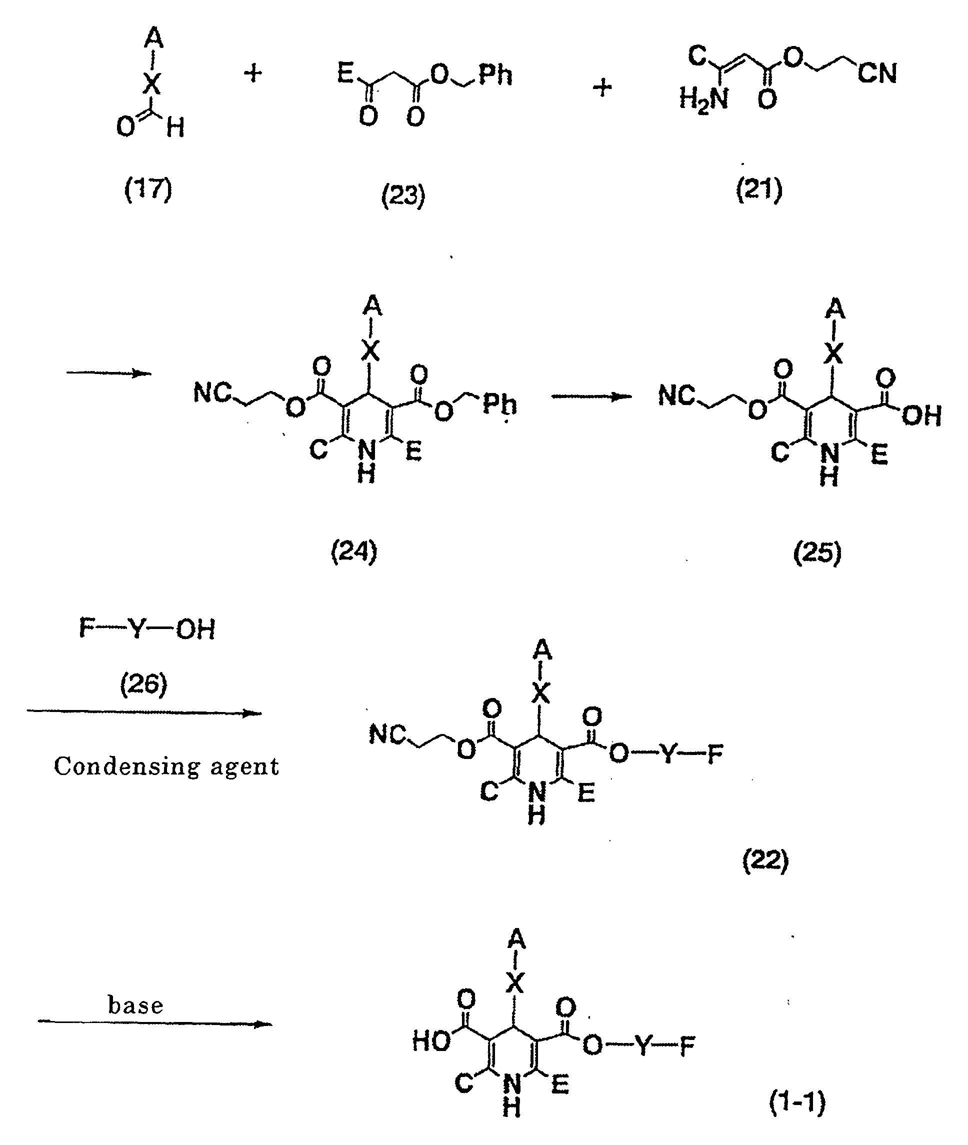

Namely, cyanoethyl esters of benzyl dihydropyridinedicarboxylates (24) can be obtained by reacting the aldehyde (17) with a benzyl ketocarboxylate (23) and 2-cyanoethyl 3-aminocrotonate having a C substituent at the 3-position. Monocyanoethyl dihydropyridinedicarboxylates (25) can be obtained by hydrogenating the obtained esters (24) in ethyl acetate in the presence of palladium catalyst. Dihydropyridinedicarboxylic acid diesters (22) can be obtained by reacting the obtained compound (25) with an alcohol (26) in the presence of a condensing agent such as WSC. Dihydropyridinecarboxylic acid derivatives (1-1) of the present invention can be produced by treating the obtained dihydropyridinedicarboxylic diesters (22) with a base such as sodium hydroxide.

Dihydropyridine derivatives (1-2) of the above formula wherein B represents carbamoyl group, nitro group or acetyl group can be produced by reacting acetoacetic acid amide (27), nitroacetone (28) or acetylacetone (29) with the aldehyde (17) and the 3-aminocrotonic acid ester (18) by the following reaction scheme: 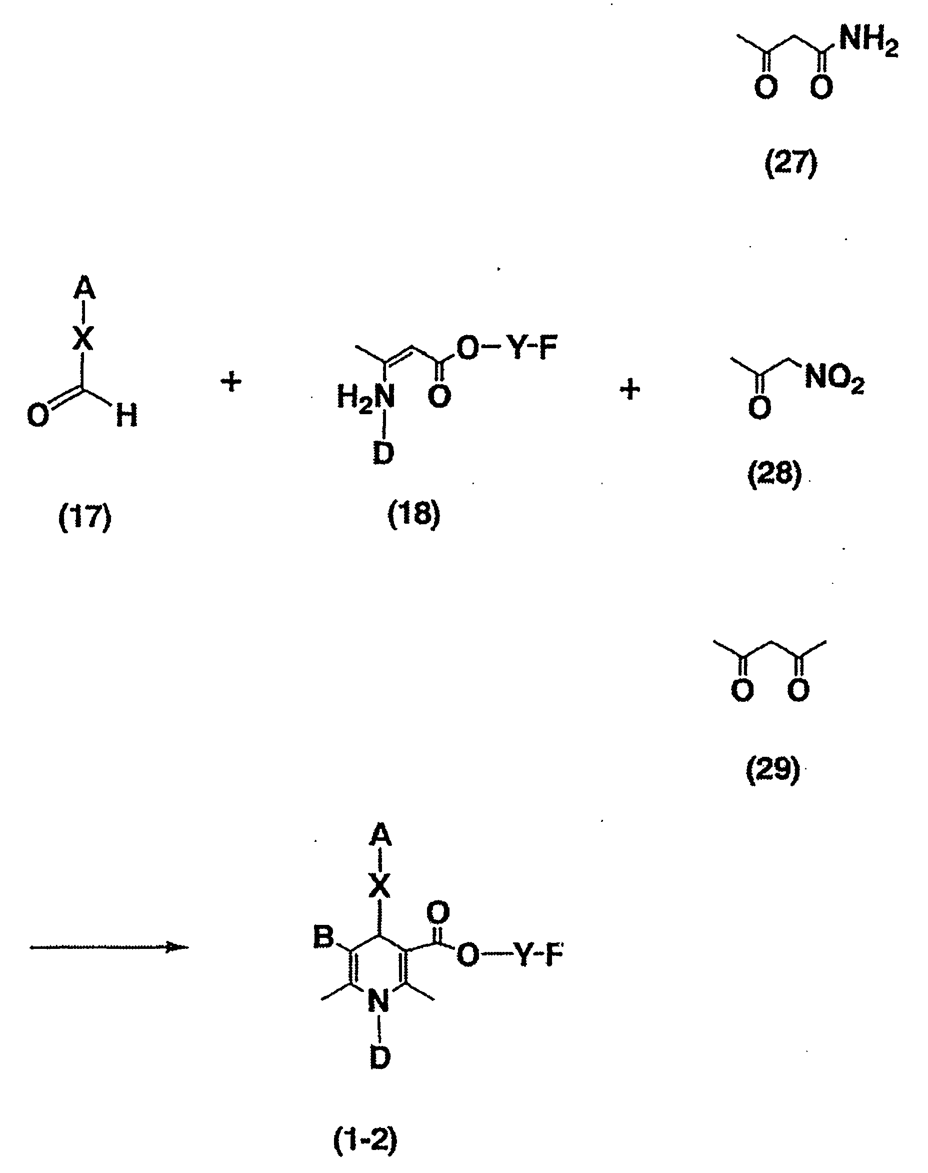 wherein A, D, F, X and Y are as defined above.

Dihydropyridine derivatives (1-3) of the above formula wherein B represents cyano group can be produced by reacting the aldehyde (17) with the acetoacetic acid ester (20) and 3-aminocrotonitrile (30) by the following reaction scheme: 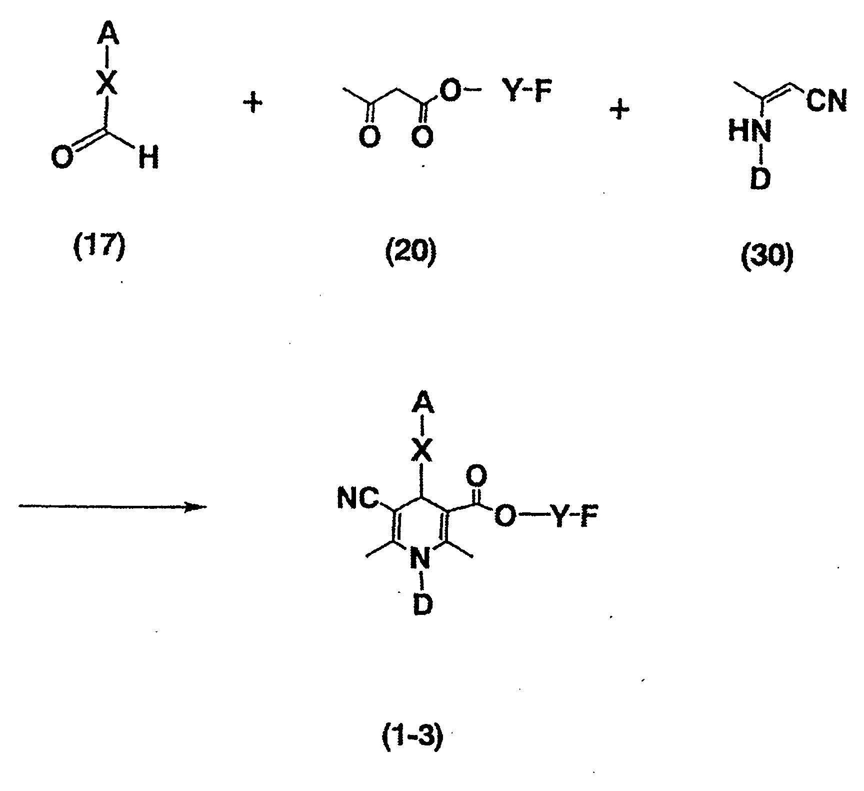 wherein A, D, F, X and Y are as defined above.

When the compounds of general formula (1) can form salts thereof, the salts are pharmaceutically acceptable ones such as ammonium salts, salts thereof with alkali metals, e. g. sodium and potassium, salts thereof with alkaline earth metals, e. g. calcium and magnesium, salts thereof with aluminum and zinc, salts thereof with organic amines, e. g. morpholine and piperidine, and salts thereof with basic amino acids, e. g. arginine and lysine.

The compounds of general formula (1) and salts thereof are administered as they are or in the form of various medicinal compositions to patients. The dosage forms of the medicinal compositions are, for example, tablets, powders, pills, granules, capsules, suppositories, solutions, sugar-coated tablets and depots. They can be prepared with ordinary preparation assistants such as carriers and diluents by an ordinary method. For example, the tablets are prepared by mixing the dihydropyridine derivative, the active ingredient of the present invention, with any of known adjuvants such as inert diluents, e. g. lactose, calcium carbonate and calcium phosphate; binders, e. g. acacia, corn starch and gelatin; extending agents, e. g. alginic acid, corn starch and pre-gelatinized starch; sweetening agents, e. g. sucrose, lactose and saccharin; corrigents, e. g. peppermint, and cherry; and lubricants, e. g. magnesium stearate, talc and carboxymethyl cellulose.

The N-type calcium channel inhibitor containing one of the compounds of above general formula (1) or one of salts thereof as active ingredient is usable as a remedy for various diseases, for example, acute stage of ischemic cerebrovascular disorders caused by cerebral infarction or intracerebral bleeding (including subarachnoidal hemorrhage); progressive neurodegenerative diseases such as Alzheimer's disease, AIDS related dementia and Parkinson's disease, dementia due to cerebrovascular disorder and ALS; neuropathy caused by head injury; pain caused by spinal injury, diabetes or thromboangiitis obliterans, postoperative pain, migraine and visceral pain; various diseases caused by psychogenic stress such as bronchial asthma, unstable angina and irritable colitis; emotional disorder withdrawal symptoms after addiction to drugs such as ethanol withdrawal symptoms.

The dose of the compound of general formula (1) or salt thereof used for the above-described purpose varies depending on the intended therapeutic effect, administration method, period of the treatment, and age and body weight of the patient. The dose is usually 1 *µ* g to 5 g a day for adults in the oral administration, and 0.01*µ*g to 1 g a day for adults in the parenteral administration.

The following Examples will further illustrate the present invention, which are only preferred embodiments of the invention and which by no means limit the invention.

### Example 1 Mono(2-(4-benzhydrylpiperazine-1-yl)ethyl) 2,6-dimethyl-4-(3-nitrophenyl)-1,4-dihydropyridine-3,5-dicarboxylate:

### 1) Synthesis of 3-(2-(4-benzhydrylpiperazine-1-yl)ethyl) 5-(2-cyanoethyl) 2,6-dimethyl-4-(3-nitrophenyl)-1,4-dihydropyridine-3,5-dicarboxylate:

774 mg (2.03 mmol) of (2-(4-benzhydrylpiperazine-1-yl)ethyl) acetoacetate, 312 mg (2.03 mmol) of 2-cyanoethyl 3-aminocrotonate and 310 mg (2.05 mmol) of 3-nitrobenzaldehyde were heated at 80°C under stirring in 20 ml of 2-propanol overnight. 2-Propanol was evaporated under reduced pressure, and the residue was purified by the silica gel chromatography (hexane / ethyl acetate: 2/1) to obtain the title compound.
Yield: 695 mg (1.07 mmol) (52.7 %) MS (ESI, m/z) 650 (M+H)⁺
¹ H-NMR (CDCl ₃ ) : 2.37 (3H, s), 2.38 (3H, s), 2.24-2.52 (8H, m), 2.56-2.66 (4H, m), 4.08-4.32 (5H, m), 5.08 (1H, s), 5.84 (1H, s), 7.14-7.44 (11H, m), 7.68 (1H, d), 7.96-7.99 (1H, m), 8.08 (1H, t)

### 2) Synthesis of mono(2-(4-benzhydrylpiperazine-1-yl)ethyl) 2,6-dimethyl-4-(3-nitrophenyl)-1,4-dihydropyridine-3,5-dicarboxylate:

687 mg (1.06 mmol) of 3-(2-(4-benzhydrylpiperazine-1-yl)ethyl) 5-(2-cyanoethyl) 2,6-dimethyl-4-(3-nitrophenyl)-1,4-dihydropyridine-3,5-dicarboxylate was dissolved in 20 ml of methanol. 2.1 ml of 1 N aqueous sodium hydroxide solution was added to the obtained solution, and they were stirred at room temperature for 5.5 hours. 2 N hydrochloric acid was added to the obtained mixture, and then methanol was evaporated under reduced pressure. Water was added to the residue, and the resultant solid was taken by the filtration, washed with water and then with a mixture of hexane and ethyl acetate (3:1), and dried under reduced pressure to obtain the title compound.
Yield: 407 mg (0.68 mmol) (64.3 %)
MS (ESI, m/z) 597 (M+H)⁺
¹ H-NMR (DMSO-d ₆ ) : 2.26 (3H, s), 2.30 (3H, s), 2.10-2.65 (10 H, m), 3.98-4.34 (3H, m), 4.98 (1H, s), 7.16-7.26 (2H, m), 7.28-7.51 (9H, m), 7.62 (1H, d), 7.91 (1H, d), 7.96 (1H, s), 8.99 (1H, s)

The structural formula of the compound obtained in Example 1 was as follows:

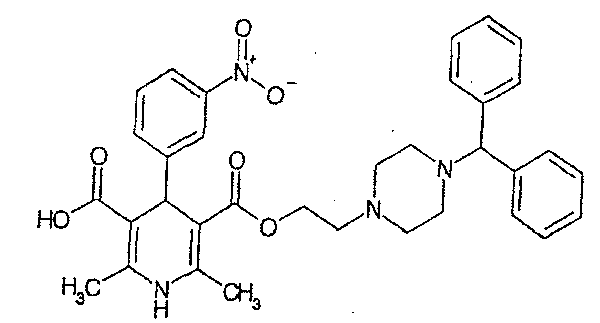

### Example 2

The inhibiting activity of the compound obtained in Example 1 on N-type calcium channel and L-type calcium channel was determined. It exhibited the activity of selectively inhibiting N-type calcium channel. Thus, the compounds of the present invention are usable as remedies for various diseases such as acute stage of ischemic cerebrovascular disorders caused by cerebral infarction or intracerebral bleeding and Alzheimer's disease.

## Claims

1. Dihydropyridine derivatives of the following general formula (1) and pharmaceutically acceptable salts thereof. 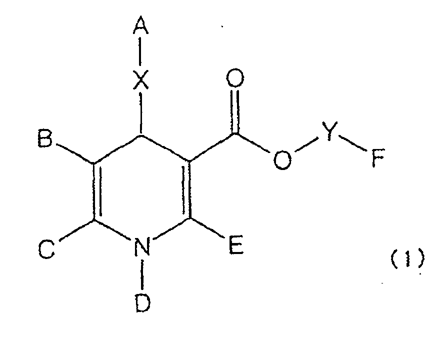 wherein A represents a group of the following general formula (2), or 1-naphthyl, 2-naphthyl, thiophene-3-yl, thiophene-2-yl, furan-3-yl, furan-2-yl, pyridine-4-yl, pyridine-3-yl, pyridine-2-yl, indole-2-yl, indole-3-yl, quinoline-2-yl, quinoline-3-yl, quinoline-4-yl, quinoline-5-yl, quinoline-6-yl, quinoline-7-yl, quinoline-8-yl, cyclohexyl or cyclopentyl group: 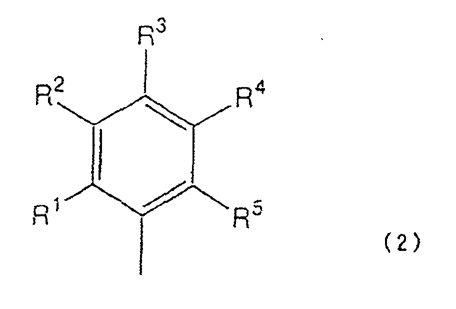 wherein R¹, R², R³, R⁴ and R⁵ may be the same or different from each other and each represent a hydrogen atom, a halogen atom, hydroxyl group, carboxyl group, amino group, cyano group, nitro group, a lower alkyl group, a lower alkoxyl group, a lower alkenyl group, a lower alkynyl group, a lower alkylamino group, a lower alkylthio group, a lower alkanoyl group, a hydroxy-lower alkyl group, a hydroxy-lower alkoxyl group, a hydroxy-lower alkenyl group, a halogeno-lower alkyl group, a halogeno-lower alkoxyl group, a halogeno-lower alkenyl group, an aryl-lower alkoxyl group, a lower-alkoxycarbonyl group, an aryl group, a heteroaryl group or an aroyl group,
B represents carbamoyl group, cyano group, nitro group, acetyl group or carboxyl group,
C represents a hydrogen atom, methyl group, ethyl group or dimethoxymethyl group,
D represents a hydrogen atom, a lower alkyl group, a hydroxy-lower alkyl group or an aryl-lower alkyl group,
E represents a hydrogen atom, methyl group, ethyl group, dimethoxymethyl group or cyano group,
F represents a heterocyclic group or a cycloalkyl group,
X represents an interatomic bond, -CH₂-, -CH₂CH₂-, -CH=CH- or -C=C-, and
Y represents an interatomic bond, -CH₂- or a group of any of the following general formulae (3) to (15): 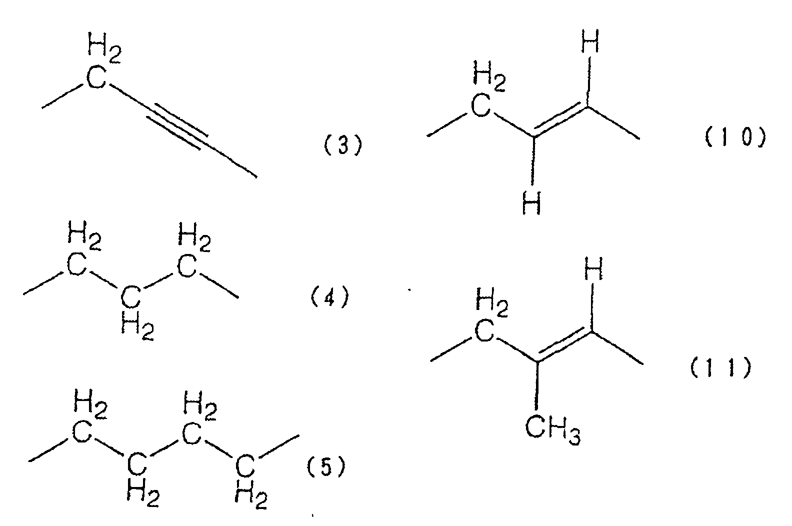 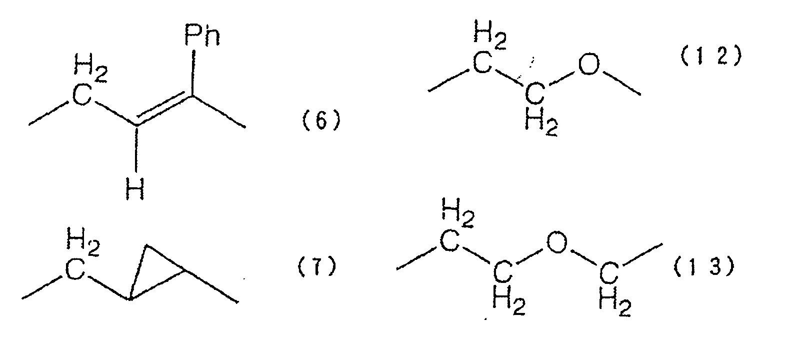 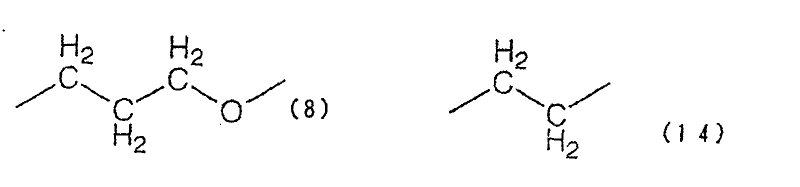 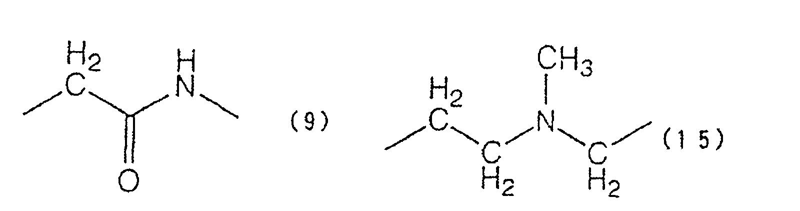
with the proviso that when Y represents any of the groups of the general formulae (3) to (15), the heterocyclic groups represented by F exclude groups of the following general formula (16), cyclohexyl group, thiophene-3-yl group, thiophene-2-yl group, furan-3-yl group, furan-2-yl group, pyridine-4-yl group, pyridine-3-yl group and pyridine-2-yl group: 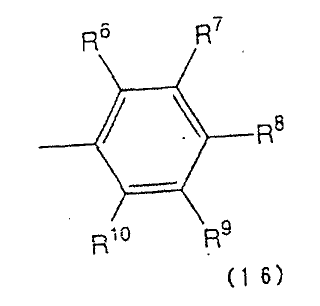 wherein R⁶, R⁷, R⁸, R⁹ and R¹⁰ may be the same or different from each other, and each represent hydrogen atom, a halogen atom, hydroxyl group, carboxyl group, amino group, cyano group, nitro group, a lower alkyl group, a lower alkoxyl group, a lower alkenyl group, a lower alkynyl group, a lower alkylamino group, a lower alkylthio group, a lower alkanoyl group, a hydroxy-lower alkyl group, a hydroxy-lower alkoxyl group, a hydroxy-lower alkenyl group, a halogeno-lower alkyl group, a halogeno-lower alkoxyl group, a halogeno-lower alkenyl group, an aryl-lower alkoxyl group, a lower-alkoxycarbonyl group or an aroyl group, and two of R¹ through R³ in general formula (2) may be bonded to each other to form a ring.

2. The dihydropyridine derivatives and pharmaceutically acceptable salts thereof according to claim 1, wherein the heterocycle represented by F is any of pyrrolidine, piperazine, pyrazolidine, imidazolidine, tetrahydrofuran, tetrahydropyran, dioxane, tetrahydrothiophene, morpholine, imidazole, pyrrolidinone, oxazole, isoxazole, pyrimidine, pyrazine, pyridazine and piperidine.

3. The dihydropyridine derivatives and pharmaceutically acceptable salts thereof according to claim 2, wherein R¹, R², R³, R⁴ and R⁵ in general formula (2), which may be the same or different from each other, represent hydrogen atom, a halogen atom, hydroxyl group, carboxyl group, cyano group, nitro group, a lower alkyl group, a lower alkoxyl group, a halogeno-lower alkyl group or a lower-alkoxycarbonyl group.

4. The dihydropyridine derivatives and pharmaceutically acceptable salts thereof according to claim 3, wherein D represents hydrogen atom, X represents an interatomic bond, and Y represents an interatomic bond, methylene group, ethylene group or propylene group.

5. The dihydropyridine derivatives and pharmaceutically acceptable salts thereof according to claim 4, wherein B represents carboxyl group.

6. The dihydropyridine derivatives and pharmaceutically acceptable salts thereof according to claim 5, wherein R¹, R², R³, R⁴ and R⁵ in general formula (2), which may be the same or different from each other, represent hydrogen atom, a halogen atom, carboxyl group, cyano group, nitro group or a halogeno-lower alkyl group, C represents methyl group, E represents methyl group and F represents any of pyrrolidine group, piperazine group, imidazole group, pyrrolidinone group or piperidine group.

7. The dihydropyridine derivatives and pharmaceutically acceptable salts thereof according to claim 5, wherein A is represented by general formula (2), wherein R¹, R³, R⁴ and R⁵ each represent hydrogen atom and R² represents nitro group, C represents methyl group, E represents methyl group and F represents piperidine group or piperazine group.

8. A N-type calcium channel antagonist containing a dihydropyridine compound or a pharmaceutically acceptable salt thereof according to claim 1 as an active ingredient.

9. The N-type calcium channel antagonist according to claim 8, wherein the heterocycle represented by F is any of pyrrolidine, piperazine, pyrazolidine, imidazolidine, tetrahydrofuran, tetrahydropyran, dioxane, tetrahydrothiophene, morpholine, imidazole, pyrrolidinone, oxazole, isoxazole, pyrimidine, pyrazine, pyridazine and piperidine.

10. The N-type calcium channel antagonist according to claim 9, wherein R¹, R², R³, R⁴ and R⁵ in general formula (2), which may be the same or different from each other, represent hydrogen atom, a halogen atom, hydroxyl group, carboxyl group, cyano group, nitro group, a lower alkyl group, a lower alkoxyl group, a halogeno-lower alkyl group or a lower-alkoxycarbonyl group.

11. The N-type calcium channel antagonist according to claim 10, wherein D represents hydrogen atom, X represents an interatomic bond, and Y represents an interatomic bond, methylene group, ethylene group or propylene group.

12. The N-type calcium channel antagonist according to claim 11, wherein, B represents carboxyl group.

13. The N-type calcium channel antagonist according to claim 12, wherein R¹, R², R³, R⁴ and R⁵ in general formula (2), which may be the same or different from each other, represent hydrogen atom, a halogen atom, carboxyl group, cyano group, nitro group or a halogeno-lower alkyl group, C represents methyl group, E represents methyl group and F represents any of pyrrolidine group, piperazine group, imidazole group, pyrrolidinone group and piperidine group.

14. The N-type calcium channel antagonist according to claim 12, wherein A is represented by general formula (2), wherein R¹, R³, R⁴ and R⁵ each represent hydrogen atom and R² represents nitro group, C represents methyl group, E represents methyl group, and F represents piperidine group or piperazine group.

15. A therapeutic agent, containing the dihydropyridine compound or the pharmaceutically acceptable salt thereof according to claim 1 as the active ingredient, for any of acute stage of ischemic cerebrovascular disorders caused by cerebral infarction or intracerebral bleeding, Alzheimer's disease, AIDS related dementia, Parkinson's disease, progressive neurodegenerative diseases, dementia due to cerebrovascular disorder, pain caused by thromboangiitis obliterans, postoperative pain, migraine, visceral pain, bronchial asthma, unstable angina, irritable colitis and withdrawal symptoms after addiction to drugs.

16. The therapeutic agent according to claim 15, wherein the heterocycle represented by F is any of pyrrolidine, piperazine, pyrazolidine, imidazolidine, tetrahydrofuran, tetrahydropyran, dioxane, tetrahydrothiophene, morpholine, imidazole, pyrrolidinone, oxazole, isoxazole, pyrimidine, pyrazine, pyridazine and piperidine.

17. A pharmaceutical composition containing any of dihydropyridine compounds and pharmaceutically acceptable salts thereof according to any of claims 1 to 7 as an active ingredient.
